# EUROPÄISCHE PATENTSCHRIFT

(11) **EP 0 545 209 B1**
(45) Veröffentlichungstag und Bekanntmachung des Hinweises auf die Patenterteilung: **03.07.1996**
(21) Anmeldenummer: 92119930.3
(22) Anmeldetag: 24.11.1992
(51) Int. Cl.: C08F 226/10, C08F 6/12, C08F 220/04, A61K 7/06

(54) **Redispergierbares Dispersionspulver aus N-Vinylpyrrolidon-Vinylacetat-Copolymerisat, dessen Herstellung und Verwendung**
Redispersable dispersion powders of a copolymer of N-vinylpyrrolidone and vinyl acetate, their preparation and use
Poudres de dispersion redispersable d'un copolymère de N-vinylpyrrolidone et d'acétate de vinyle, leur préparation et usage

(30) Priorität: 04.12.1991 DE 4139963
(43) Veröffentlichungstag der Anmeldung: 09.06.1993
(73) Patentinhaber: BASF Aktiengesellschaft, D-67063 Ludwigshafen (DE)
(72) Erfinder: Buehler, Volker, Dr., W-6706 Wachenheim (DE); Grabowski, Sven, Dr., W-6700 Ludwigshafen (DE); Sanner, Axel, Dr., W-6710 Frankenthal (DE)

(56) Entgegenhaltungen:
- EP-A- 0 418 721
- CH-A- 330 141

## Beschreibung

### Beschreibung

Die Erfindung betrifft ein Verfahren zur Herstellung eines rieselfähigen, redispergierbaren Dispersionspulvers aus einem wasserunlöslichen Vinylpyrrolidon (VP)-Vinylacetat (VAc)-Copolymeren durch Copolymerisation einer Mischung der Monomeren in organischer Lösung, wobei die so entstandene Lösung nach Zusatz eines Tensids einem Lösungsmittelaustausch gegen Wasser unterworfen und die so erhaltene Dispersion sprüh- oder gefriergetrocknet wird, das nach diesem Verfahren erhältliche Pulver als solches und seine Verwendung, vor allem zur gesteuerten Wirkstofffreisetzung.

Vinylpyrrolidon-Vinylacetat-Lösungspolymere mit einem Gehalt von mehr als 50 Gew.-% Vinylacetat sind wasserunlöslich und werden für Überzüge und Beschichtungen als Lösung in organischen Lösungsmitteln eingesetzt. Der Einsatz von organischen Lösungsmitteln ist bekanntlich problematisch; die verhältnismäßig teuren organischen Lösungsmittel, die sich nur schwer, in der Regel gar nicht zurückgewinnen lassen und daher die Umwelt belasten, führen zu einer Feuer- und Explosionsgefährdung und sind auch physiologisch nicht unbedenklich. Eine Alternative zu diesen Lösungspolymerisaten böte lediglich die Anwendung der entsprechenden Dispersionen. Aus den US-Patentschriften 3 244 658, 3 691 125 und 4 167 439 ist bekannt, daß Emulsionspolymerisationen mit Vinylpyrrolidon als Comonomer (mehr als 10 Gew.-% der Monomeren) zu instabilen, sich trennenden, hochviskosen Dispersionen führen, die sich zudem schlecht reproduzieren lassen. Lediglich durch Pfropfung von Vinylacetat auf in der Emulsionspolymerisation vorgelegtes Polyvinylpyrrolidon (PVP) lassen sich derartige Dispersionen gewinnen (US 3 691 125). Eine Sekundärdispersion eines derartigen Pfropfcopolymeren aus Vinylestern auf Polyvinylpyrrolidon ist in der DE-A 15 44 860 beschrieben. Das Herstellverfahren ist aber sehr umständlich, das Pfropfcopolymer wird zunächst verseift, mit Diketen umgesetzt, ausgefällt, mit Wasser gewaschen und anschließend in einem mit Wasser mischbaren Lösungsmittel gelöst oder emulgiert, dann in Wasser dispergiert, schließlich wird das Lösungsmittel abdestilliert.

Ganz abgesehen von der Kompliziertheit dieses Verfahrens ist das so erhaltene Pfropfcopolymere nicht vergleichbar mit einem Lösungspolymerisat der gleichen Comonomeren in gleichen Mengenverhältnissen.

Aus der EP-A 418 721 sind in Wasser klar lösliche Copolymerisate aus 45 bis 90 Gew.-% N-Vinylpyrrolidon und 10 bis 55 Gew.-% eines Vinylesters bekannt. Diese Copolymerisate werden durch radikalische Polymerisation in einem organischen Lösungsmittel erhalten, wobei das Lösungsmittel zunächst destillativ abgetrennt wird und weitere flüchtige organische Bestandteile danach durch Wasserdampfdestillation entfernt werden.

Der Erfindung lag die Aufgabe zugrunde, in einfacher Weise ein redispergierbares, rieselfähiges Dispersionspulver eines wasserunlöslichen Vinylpyrrolidon-Vinylacetat-Lösungspolymerisates herzustellen, das durch Anrühren mit oder Einrühren in kaltes Wasser eine wäßrige Zubereitungsform des genannten Polymeren liefert.

Es wurde nun gefunden, daß sich aus organischen Lösungen wasserunlöslicher Vinylpyrrolidon-Vinylacetat-Lösungscopolymerisate, zweckmäßig direkt aus den Polymerisationslösungen, durch Zugabe von einem oder mehreren Tensiden oder Emulgatoren und gegebenenfalls Schutzkolloiden und einem anschließenden Lösungsmittelaustausch gegen Wasser eine stabile Sekundärdispersion herstellen läßt, aus der sich durch Gefrier- oder Sprühtrocknen gut redispergierbare, rieselfähige Dispersionspulver gewinnen lassen. Das Copolymer wird in üblicher Weise hergestellt aus 15 bis 40, vorzugsweise 20 bis 35 Gew.-% Vinylpyrrolidon und 60 bis 85, vorzugsweise 65 bis 80 Gew.-% Vinylacetat in einem organischen Lösungsmittel (oder Lösungsmittelgemisch) mit einem Radikalstarter. Vorteilhaft lassen sich Lösungspolymerisate mit bereits sehr geringem Restmonomerengehalt nutzen, wie sie in US 4 182 851 beschrieben sind. Der K-Wert nach Fikentscher (Cellulose-Chemie 13 (1932), Seiten 58 bis 64 und 71 bis 74) des Polymeren soll im Bereich von 16 bis 36, vorzugsweise 25 bis 36, liegen, gemessen in 1 %iger ethanolischer Lösung bei 25°C.

Das Tensid wird entsprechend den Erfordernissen der späteren Verwendung der Dispersion aus den üblichen anionischen, kationischen oder nichtionischen Tensiden ausgewählt. Für den Einsatz der Dispersion in der pharmazeutischen Industrie haben sich beispielsweise folgende Tenside sehr gut bewährt: Natriumsalz des Sulfobernsteinsäureethylhexylesters, Arylsulfonate, ca. 9fach oxethylierte Stearinsäure und Natriumlaurylsulfat.

Als Schutzkolloid eignen sich alle bei der Emulsionspolymerisation als Schutzkolloid verwendbaren Verbindungen, beispielsweise wasserlösliche Cellulosederivate, Gelatine oder PVP. Sie müssen in Wasser entweder echt oder kolloidal löslich sein.

Der Lösungsmittelaustausch kann z.B. durch Wasserdampfdestillation unter Normaldruck oder durch schrittweise Zugabe von Wasser und anschließendes Entfernen des organischen Lösungsmittels unter reduziertem Druck erfolgen und sollte die möglichst vollständige Entfernung des organischen Lösungsmittels zur Folge haben.

Als organisches Lösungsmittel bei der Polymerisation kommen z.B. aliphatische Ketone mit 3 bis 5 Kohlenstoffatomen, Toluol und vor allem niedere aliphatische Alkohole, insbesondere Ethanol oder Isopropanol oder deren Mischungen, in Betracht.

Die Trocknung der Sekundärdispersion kann durch Gefriertrocknung oder durch Sprühtrocknung, ggf. unter Zusatz von Sprühhilfsmitteln und Antiblockmitteln, erfolgen. Die Sprühtrocknung erfolgt in an sich bekannter Weise in Sprühtürmen, wobei die zu trocknende Dispersion mit Hilfe von Zerstäubungsscheiben oder Ein- bzw. Mehrstoffdüsen eingesprüht werden kann. Die Trocknung der Dispersion wird mit heißen Gasen, z.B. mit Stickstoff oder Luft, durchgeführt.

Als Sprühhilfsmittel werden eine oder mehrere wasserlösliche Substanzen mit einem Phasenumwandlungspunkt zweiter Ordnung (Glasübergangstemperatur Tg) bei mindestens 60°C eingesetzt, und zwar in Mengen von 0 bis 50 Gew.-% des Polymerisates. Als derartige Verdüsungshilfe haben sich vor allem polymere wasserlösliche Substanzen bewährt, insbesondere solche mit hohen Polymerisationsgraden. Als Beispiele seien genannt: Polyvinylalkohole, Ligninsulfonate, wasserlösliche Kondensationsprodukte aus Naphthalinsulfonsäure und Formaldehyd, Polyacrylsäuren und Polyacrylamide.

Zur Erhöhung der Lagerfähigkeit, um z.B. bei Dispersionspulvern mit niedriger Glasübergangstemperatur ein Verbacken und Verblocken zu verhindern und somit die Redispergierbarkeit zu verbessern, kann das erhaltene Pulver mit 0 bis 30 Gew.-%, bezogen auf das Gesamtgewicht polymerer Bestandteile, an Antiblockmitteln versetzt werden. Alle für derartige Zwecke üblichen Antiblockmittel sind geeignet, beispielsweise kolloidales Kieselgel, Talkum, gemahlene Tone und Calciumcarbonat.

Das redispergierbare Dispersionspulver kann bis zur Bereitung der als Überzugsmittel genutzten Dispersion raum- und gewichtssparend transportiert und ohne Gefahr eines Qualitätsverlustes als trockenes Pulver gelagert werden. Die Eigenschaften des Lösungspolymerisates bleiben im dispergierten Zustand im wesentlichen erhalten. So gleichen die beim Trocknen der organischen Lösung des Polymeren erhaltenen Filme im wesentlichen denen, die man nach Trocknung der Sekundärdispersion erhält, sofern bei der Herstellung der Sekundärdispersion nur geringe Hilfsstoffmengen erforderlich waren. Darüber hinaus führt das Verfahren des Lösungsmittelaustausches, das einem "Strippen" der Dispersion mit Wasserdampf gleicht, zur Verminderung unerwünschter flüchtiger niedermolekularer Bestandteile. Dadurch kann insbesondere der Vinylacetat-Restmonomerengehalt gesenkt und eine Geruchsverringerung gegenüber dem Lösungspolymerisat erzielt werden.

Die nach dem erfindungsgemäßen Verfahren erhältlichen Dispersionspulver lassen sich durch einfaches Anrühren mit kaltem Wasser redispergieren, wobei die so erhaltene Dispersion im wesentlichen die gleichen Eigenschaften wie die ursprünglich beim Lösungsmittelaustausch erhaltene Sekundärdispersion besitzt, sofern keine größeren Mengen Hilfsmittel zugesetzt worden waren. Sie lassen sich mit Vorteil z.B. im Bausektor, bei der Herstellung von Anstrichs- und Beschichtungsmitteln, Leimen und Klebstoffen einsetzen. Besonders bewährt haben sie sich als Binde- und Überzugsmittel für pharmazeutische Retardformen mit pH-Wert-unabhängiger Steuerung der Freisetzung des Wirkstoffs sowie als Filmbildner für Haarsprayformulierungen.

Feste pharmazeutische Formen mit kontrollierter Abgabe des Wirkstoffs (Retardformen) werden meist unter Verwendung eines Polymeren als Matrixtabletten, Filmtabletten oder überzogene Pellets bzw. Granulate mit oder ohne Hartgelatinekapselhülle vermarktet. Als Polymer werden üblicherweise Cellulosederivate, Schellack oder Methacrylcopolymere verwendet (H. Sucker, P. Fuchs, P. Speiser, Pharmazeutische Technologie, G. Thieme-Verlag Stuttgart, 1978, S. 349 - 354). Diese Polymere umhüllen den Wirkstoff und setzen ihn im Magen- und/oder Darmsaft nach dem Prinzip der langsamen Diffusion durch das Polymer und/oder Erosion des Polymers in Abhängigkeit des pH-Wertes oder unabhängig davon frei. Ein Ziel muß dabei sein, am Anfang eine ausreichende Freisetzung zu erhalten, um die Mindestwirkstoffkonzentration im Blut zu erreichen, worauf die Phase einer langsameren Wirkstoffabgabe folgen muß.

Die Techniken der Anwendung der erfindungsgemäß erhältlichen Pulver und Dispersionen sind die in der Galenik üblichen:

Zur Herstellung von Matrixtabletten oder Granulaten kann die Technologie der traditionellen Feuchtgranulation des Wirkstoffs im Kneter/Mischer oder die Wirbelschichtgranulation angewandt werden. Hierbei wird die wäßrige Dispersion des erfindungsgemäß erhältlichen Pulvers als Bindemittelsuspension verwendet. Als Alternative kann der pharmazeutische Wirkstoff mit dem erfindungsgemäß erhältlichen Dispersionspulver gemischt werden und diese Mischung mit einem Lösungsmittel, bevorzugt Wasser oder auch Alkohole, mit den obengenannten Technologien granuliert werden. Auch eine Kombination beider Verfahren kann in manchen Fällen sinnvoll sein. Unter Umständen empfiehlt sich der Zusatz eines Füllmittels zum Wirkstoff (z.B. Lactose, Stärke, Calciumhydrogenphosphat) und/oder eines Antiklebemittels (z.B. Talkum). Nach Siebung und Trocknung des feuchten Granulats kann dieses entweder als solches verpackt oder in Hartgelatinekapseln gefüllt oder nach Zusatz von weiteren Tablettierhilfsmitteln (z.B. Schmiermitteln) zu Tabletten verpreßt werden.

Zur Herstellung von Pellets kann der Wirkstoff in der wäßrigen Dispersion des erfindungsgemäß erhältlichen Pulvers gelöst oder fein dispergiert werden. Anschließend erfolgt die Beschichtung von Trägermaterialteilchen (z.B. Zuckerperlen) mit dieser Dispersion in üblicher Weise, z.B. im Wirbelbett, bis die gewünschte Wirkstoffmenge aufgetragen ist. Ein weiteres mehr oder weniger starkes Beschichten der Pellets mit der Dispersion des erfindungsgemäß erhältlichen Pulvers ohne Wirkstoff kann zur zusätzlichen Steuerung der Wirkstofffreisetzung aus den Pellets dienen.

Zur Herstellung der erfindungsgemäßen Filmtabletten wird eine wäßrige Dispersion (oder auch eine organische, vorzugsweise alkoholische, Lösung) des erfindungsgemäß erhältlichen Pulvers zusammen mit den für Tablettenüberzüge üblichen Zusatzstoffen (z.B. Pigmente, Lacke, Talkum) im Wirbelbett, Dragierkessel, Accela-Cota oder vergleichbaren Apparaten auf die wirkstoffhaltigen Drageekerne aufgetragen.

Man erhält auf diese Weise eine pH-Wert-unabhängige Steuerung der Wirkstofffreisetzung, die mit der anderer bisher üblicher Retard-Filmbildner vergleichbar ist. Durch Variation des verwendeten Vinylpyrrolidon-Vinylacetat-Mengenverhältnisses läßt sich das Freisetzungsprofil des Wirkstoffs steuern.

### Beispiel 1

Ein Copolymerisat aus 30 Gew.-% Vinylpyrrolidon und 70 Gew.-% Vinylacetat (hergestellt durch Lösungspolymerisation in Isopropanol, K-Wert nach Fikentscher 28 + 3, gemessen an einer 1 % Lösung des Polymeren in Ethanol bei 25°C) wurde als 50%ige Lösung in Isopropanol mit 0,1 Gew.-% (bezogen auf das Polymer) Natriumlaurylsulfat versetzt und unter Rühren so lange wasserdampfdestilliert, bis alles Isopropanol gegen Wasser ausgetauscht war. Man erhielt eine weiße Dispersion, deren Feststoffgehalt auf 30 % eingestellt wurde. Die Viskosität der Dispersion betrug 159 mPas, der Restmonomerengehalt an Vinylpyrrolidon und Vinylacetat war kleiner als 2 ppm, bezogen auf das Polymer. Eine weitere Stabilisierung dieser Dispersion wurde durch Zugabe von etwa 1 Gew.-% 9fach ethoxylierter Stearinsäure erreicht.

Die auf ca. 20 % Feststoffgehalt eingestellte Dispersion wurde gefriergetrocknet und der Trockenrückstand zu einem feinen, rieselfähigen, nichtblockenden Pulver verrieben. Es zeigte nach mehrmonatiger Lagerung bei ca. 25°C kein Verbakken.

### Beispiel 2

Ein Copolymerisat aus 30 Gew.-% Vinylpyrrolidon und 70 Gew.-% Vinylacetat (hergestellt durch Lösungspolymerisation in Ethanol, K-Wert 32 ± 4, Messung wie bei Beispiel 1) wurde als 50%ige Lösung in Ethanol mit 0,1 Gew.-%, bezogen auf das Polymer, Natriumlaurylsulfat versetzt und entsprechend Beispiel 1 behandelt. Die 30%ige Dispersion wies eine Viskosität von 4200 mPas auf. Sie wurde nach Verdünnung mit Wasser auf das doppelte Volumen sprühgetrocknet. Die Eingangstemperatur betrug ca. 120, die Ausgangstemperatur etwa 80°C. Es fiel ein gut rieselfähiges, nichtblockendes, feines Sprühprodukt an, bestehend aus agglomerierten Teilchen relativ einheitlicher Struktur. Es zeigte nach mehrmonatigem Lagern bei ca. 25°C kein Verbacken.

### Beispiel 3

Ein Copolymerisat aus 20 Gew.-% Vinylpyrrolidon und 80 Gew.-% Vinylacetat (hergestellt durch Lösungspolymerisation in Isopropanol, K-Wert 19 ± 3, Messung wie bei Beispiel 1) wurde als 50%ige Lösung in Isopropanol mit 1,6 Gew.-%, bezogen auf das Polymer, Natriumlaurylsulfat versetzt und entsprechend Beispiel 1 einer Wasserdampfdestillation unterzogen. Die auf 25 Gew.-% eingestellte Dispersion wurde durch anschließende Zugabe von 1 Gew.-% des Natriumsalzes des Sulfobernsteinsäureethylhexylesters stabilisiert. Nach Verdünnen mit Wasser auf ca. 15 % Festgehalt wurde sprühgetrocknet. Die Eingangstemperatur betrug etwa 105, die Ausgangstemperatur ca. 70°C. Das erhaltene Pulver hatte ähnliche Eigenschaften wie das von Beispiel 2.

Die gemäß den Beispielen 1 bis 3 erhaltenen Pulver ließen sich durch Einrühren in kaltes Wasser leicht dispergieren. Die so erhaltenen Dispersionen (mit praktisch beliebig einstellbarer Feststoffkonzentration) ergaben nach dem Trocknen einheitliche Filme, die sich hinsichtlich ihrer Transparenz und Sprödigkeit praktisch nicht von denen aus einer organischen Lösung des Lösungspolymerisates unterschieden. Die Mindestfilmbildetemperatur (nach DIN 53 787) dieser Dispersionen betrug 14 bis 17°C.

### Beispiel 4

Mit Hilfe einer wäßrigen Dispersion des gemäß Beispiel 1 erhaltenen Pulvers wurden Matrixtabletten nach folgender Formulierung hergestellt:

### 1. Zusammensetzung

| | | |
|---|---|---|
| I | Theophyllin | 125 g |
| | Calciumhydrogenphosphat | 75 g |
| | | |
| II | Vinylpyrrolidon-Vinylacetat-Copolymer | |
| | Dispersion 30 % | 33 g |
| | Wasser | 27 g |
| | | |
| III | Magnesiumstearat | 1 g |

Die Mischung I wurde im Wirbelschichtgranulator mit Dispersion II granuliert, gesiebt, getrocknet, mit III gemischt und auf einem Rundläufer mit niederer Preßkraft zu Tabletten gepreßt.

### 2. Physikalische Eigenschaften der Tabletten

| | |
|---|---|
| Gewicht | 213 mg |
| Durchmesser | 8 mm |
| Härte (nach Schleuniger) | 186 N |
| Abrieb (Roche-Friabilator) | 0,2 % |

### 3. Freisetzung des Theophyllins

Die Freisetzung des Theophyllins wurde im Freisetzungsgerät gemäß US-Pharmakopöe XXII, Paddle-Methode, bei 50 Umdrehungen pro Minute bestimmt. Als Vergleich wurde eine analog hergestellte Matrixtablette mit einem handelsüblichen Ethylacrylat/Methylmethacrylat-Copolymer anstelle des erfindungsgemäßen Vinylpyrrolidon-Vinylacetat-Copolymeren verwendet. Die beobachteten Freisetzungsverläufe waren einander sehr ähnlich, doch erwies sich die Matrix gemäß Beispiel 1 insofern als günstiger, als die Freisetzung am Anfang etwas höher und später etwas niedriger war als bei den Vergleichstabletten.

### Beispiel 5

Aus dem erfindungsgemäßen Dispersionspulver von Beispiel 1 wurde eine Dispersion mit 10 Gew.-% Festgehalt in destilliertem Wasser hergestellt. Diese Dispersion ließ sich sehr gut als Pump-Spray zur Haarfestigung verwenden. Der Film war klar und zeigte auch ohne Zusatz eines Verlaufhilfsmittels einen angenehmen Glanz. Für praktische Anwendungen wurden lediglich Parfumöle zugesetzt.

## Patentansprüche

1. Redispergierbares, rieselfähiges Dispersionspulver aus einem 15 bis 40 Gew.-% Vinylpyrrolidon einpolymerisiert enthaltenden wasserunlöslichen Vinylpyrrolidon-Vinylacetat-Copolymeren, erhältlich durch Copolymerisation einer Mischung der Monomeren in organischer Lösung, wobei die so entstandene Lösung nach Zusatz eines Tensids einem Lösungsmittelaustausch gegen Wasser unterworfen und die so erhaltene Dispersion sprüh- oder gefriergetrocknet wird.

2. Verfahren zur Herstellung eines redispergierbaren, rieselfähigen Dispersionspulvers nach Anspruch 1, dadurch gekennzeichnet, daß die Mischung der Monomeren in organischer Lösung copolymerisiert wird, die so entstandene Lösung nach Zusatz eines Tensids einem Lösungsmittelaustausch gegen Wasser unterworfen und die so erhaltene Dispersion sprüh- oder gefriergetrocknet wird.

3. Verfahren nach Anspruch 2, dadurch gekennzeichnet, daß man der Polymerlösung vor dem Lösungsmittelaustausch neben dem erwähnten Tensid ein Schutzkolloid zusetzt.

4. Verwendung des redispergierbaren, rieselfähigen Dispersionspulvers nach Anspruch 1 zur Herstellung von Anstrichs- und Beschichtungsmitteln, Leimen und Klebstoffen.

5. Verwendung des redispergierbaren, rieselfähigen Dispersionspulvers nach Anspruch 1 zur Herstellung von Matrix oder Überzug von festen pharmazeutischen Retardformen.

6. Verwendung des redispergierbaren, rieselfähigen Dispersionspulvers nach Anspruch 1 zur Herstellung von Haarsprayformulierungen.

## Claims

1. A free-flowing redispersible powder composed of a water-insoluble vinylpyrrolidone/vinyl acetate copolymer containing 15-40% by weight of vinylpyrrolidone units, obtainable by copolymerizing a mixture of the monomers in organic solution, where a surfactant is added to the resulting solution and then the solvent is replaced by water, and the resulting dispersion is spray or freeze-dried.

2. A process for preparing a free-flowing redispersible powder as claimed in claim 1, which comprises copolymerizing the mixture of the monomers in organic solution, adding a surfactant to the resulting solution and then replacing the solvent by water, and spray- or freeze-drying the resulting dispersion.

3. A process as claimed in claim 2, wherein, besides the said surfactant, a protective colloid is added to the polymer solution before the solvent is replaced.

4. The use of the free-flowing redispersible powder as claimed in claim 1 for producing paints and coating compositions, glues and adhesives.

5. The use of the free-flowing redispersible powder as claimed in claim 1 for producing the matrix or coating of solid slow-release pharmaceutical forms.

6. The use of the free-flowing redispersible powder as claimed in claim 1 for producing hair spray formulations.

## Revendications

1. Poudre en dispersion versable, redispersable, composée d'un copolymère de vinylpyrrolidone-acétate de vinyle insoluble dans l'eau, contenant à l'état copolymérisé 15 à 40 % en poids de vinylpyrrolidone, pouvant être obtenue par copolymérisation d'un mélange des monomères en solution organique,tandis que la solution ainsi formée est soumise après addition d'un agent de surface à un échange de solvant contre de l'eau et la dispersion ainsi obtenue est séchée par pulvérisation ou par lyophilisation.

2. Procédé pour la préparation d'une poudre en dispersion versable, redispersable, selon la revendication 1, caractérisé par le fait que le mélange des monomères est copolymérisé en solution organique, la solution ainsi formée est soumise après addition d'un agent de surface à un échange de solvant contre de l'eau et la dispersion ainsi obtenue est séchée par pulvérisation ou par lyophilisation.

3. Procédé selon la revendication 2, caractérisé par le fait qu'on ajoute un colloïde protecteur à la solution de polymère avant l'échange de solvant, en plus de l'agent de surface mentionné.

4. Utilisation de la poudre en dispersion versable, redispersable, selon la revendication 1, pour la préparation d'agents d'enduction et de revêtement, de colles et d'adhésifs.

5. Utilisation de la poudre en dispersion versable, redispersable, selon la revendication 1, pour la préparation de matrice ou d'enrobage de formes pharmaceutiques solides à effet retard.

6. Utilisation de la poudre en dispersion versable, redispersable, selon la revendication 1, pour la préparation de formulations de sprays pour cheveux.
